(19) **Europäisches Patentamt**

**European Patent Office**

**Office européen des brevets**

(11) **EP 0 668 102 B1**

(12) **EUROPÄISCHE PATENTSCHRIFT**

(45) Veröffentlichungstag und Bekanntmachung des Hinweises auf die Patenterteilung:
**12.05.1999 Patentblatt 1999/19**

(51) Int. Cl.$^6$: **B01J 23/88**, B01J 27/199

(21) Anmeldenummer: **95101741.7**

(22) Anmeldetag: **09.02.1995**

(54) **Multimetalloxidmassen**

Multimetal oxide masses

Masses d'oxydes multimétalliques

(84) Benannte Vertragsstaaten:
**BE DE ES FR GB NL**

(30) Priorität: **17.02.1994 DE 4405058**

(43) Veröffentlichungstag der Anmeldung:
**23.08.1995 Patentblatt 1995/34**

(73) Patentinhaber:
**BASF Aktiengesellschaft**
**67063 Ludwigshafen (DE)**

(72) Erfinder:
- **Martin, Friedrich-Georg, Dr.**
  **D-69115 Heidelberg (DE)**
- **Hibst, Hartmut, Dr.**
  **D-69198 Schriesheim (DE)**
- **Tenten, Andreas, Dr.**
  **D-67434 Neustadt (DE)**

(56) Entgegenhaltungen:
EP-A- 0 000 835     EP-A- 0 358 411
EP-A- 0 446 644     EP-A- 0 467 144
EP-A- 0 575 897     FR-A- 2 534 904

**Beschreibung**

Die Erfindung betrifft Multimetalloxidmassen der allgemeinen Formel I

$$[A]_p [B]_q, \hspace{6cm} (I),$$

in der die Variablen folgende Bedeutung haben:

A  $Mo_{12} X_a^1 X_b^2 X_c^3 X_d^4 S_e X_f^5 O_x$ (Co-Phase)

B  $X_{12}^6 CU_g X_h^7 O_Y$ (Schlüsselphase)

$X^1$  Phosphor, Arsen, Bor, Germanium und/oder Silicium,

$X^2$  Vanadium, Niob und/oder Wolfram,

$X^3$  Wasserstoff, von dem bis zu 97 mol-% ersetzt sein können durch Kalium, Rubidium, Caesium und/oder Ammonium ($NH_4$),

$X^4$  Antimon und/oder Wismut,

$X^5$  Rhenium und/oder Rhodium,

$X^6$  Molybdän, Wolfram, Niob und/oder Tantal,

$X^7$  Lithium, Natrium, Kalium, Rubidium, Caesium und/oder Ammonium ($NH_4$),

a  1 bis 6, vorzugsweise 1 bis 3 und besonders bevorzugt 1,5 bis 2,5,

b  0 bis 6, vorzugsweise 0,2 bis 4 und besonders bevorzugt 0,5 bis 2,

c  3 bis 5;

d  0 bis 6, vorzugsweise 0 bis 3 und besonders bevorzugt 0,5 bis 1,5,

e  0 bis 3, vorzugsweise 0,01 bis 1 und besonders bevorzugt 0,01 bis 0,2,

f  0 bis 3, vorzugsweise 0,01 bis 1 und besonders bevorzugt 0,01 bis 0,5,

g  4 bis 24, vorzugsweise 5 bis 18 und besonders bevorzugt 8 bis 15,

h  0 bis 20, vorzugsweise 0 bis 12 und besonders bevorzugt 6 bis 12,

x,y  Zahlen, die durch die Wertigkeit und Häufigkeit der von Sauerstoff verschiedenen Elemente in I bestimmt werden und

p,q  von Null verschiedene Zahlen, deren Verhältnis p/q 12:0,1 bis 12:48, vorzugsweise 12:0,25 bis 12:12 und besonders bevorzugt 12:0,5 bis 12:4 beträgt,

die den Anteil $[A]_p$ in Form dreidimensional ausgedehnter, von ihrer lokalen Umgebung aufgrund ihrer von ihrer lokalen Umgebung verschiedenen chemischen Zusammensetzung abgegrenzter, Bereiche A der chemischen Zusammensetzung

A  $Mo_{12} X_a^1 X_b^2 X_c^3 X_d^4 S_e X_f^5 O_x$ (Co-Phase)

und den Anteil $[B]_q$ in Form dreidimensional ausgedehnter, von ihrer lokalen Umgebung aufgrund ihrer von ihrer lokalen Umgebung verschiedenen chemischen Zusammensetzung abgegrenzter, Bereiche B der chemischen Zusammensetzung

B     $X^6_{12}$ $Cu_g$ $X^7_h$ $O_y$ (Schlüsselphase)

enthalten, wobei die Bereiche A, B relativ zueinander wie in einem Gemisch aus feinteiligem A und feinteiligem B verteilt sind.

Außerdem betrifft vorliegende Erfindung Verfahren zur Herstellung dieser Massen sowie deren Verwendung.

Die EP-A 446 644 betrifft Multimetalloxidmassen, deren Element-Bruttozusammensetzung derjenigen der erfindungsgemäßen Multimetalloxidmassen entspricht.

Die Herstellung dieser Multimetalloxidmassen erfolgt dadurch, daß man geeignete Quellen der Bestandteile der gewünschten Multimetalloxidmassen in den erforderlichen Mengen zu einem innigen Trockengemisch verarbeitet und dieses anschließend bei erhöhter Temperatur mehrere Stunden calciniert. Die resultierenden Multimetalloxidmassen werden u.a. als Katalysatoren zur gasphasenkatalytisch oxidativen Herstellung von Methacrylsäure aus Methacrolein empfohlen. Nachteilig an den Multimetalloxidmassen dieses Standes der Technik ist jedoch, daß sowohl ihre Aktivität als auch die Selektivität der Methacrylsäurebildung bei vorgegebenem Umsatz nicht voll zu befriedigen vermag. Desgleichen gilt für die Reproduzierbarkeit ihrer Herstellung sowie für die Standzeiten während ihres Gebrauchs. Die Standzeiten vermögen insbesondere dann nicht zu befriedigen, wenn die Methacrolein als Hauptbestandteil umfassenden Reaktionsgase als Nebenbestandteile organische Säuren beinhalten.

Die EP-A 835, die DE-C 3 338 380, die DE-A 42 20 859 und die ältere Anmeldung DE-A 43 07 381 betreffen ebenfalls als Katalysatoren für die gasphasenkatalytisch oxidative Herstellung von Methacrylsäure aus Methacrolein geeignete Multimetalloxidmassen, die in vorteilhafter Weise gleichfalls einen Schlüsselphase/Co-Phase Aufbau aufweisen. Zwar umfassen die allgemeinen Formeln dieses Standes der Technik innerhalb einer breiten Mannigfaltigkeit möglicher Multimetalloxidmassen formal auch solche, deren Schlüsselphase neben Elementen wie Molybdän oder Wolfram einerseits gleichzeitig das Element Kupfer und deren Co-Phase andererseits gleichzeitig z.B. Phosphor, Arsen oder Antimon enthalten können. Die Gesamtheit aller Ausführungsbeispiele umfaßt jedoch kein einziges solches Ausführungsbeispiel, vielmehr sind selbige auf solche beschränkt, deren Schlüsselphase anstelle des Elements Kupfer das Element Wismut enthalten. Diese Ausführungsform empfiehlt der Stand der Technik nachdrücklich als die besonders bevorzugte, insbesondere auch als Katalysator für die katalytische Gasphasenoxidation von Methacrolein zu Methacrylsäure. Nachteilig an dieser bevorzugten Ausführungsform des Standes der Technik ist jedoch, daß auch sie als Katalysator für die katalytische Gasphasenoxidation von Methacrolein zu Methacrylsäure nicht voll zu befriedigen vermag.

Aufgabe der vorliegenden Erfindung war daher, Multimetalloxidmassen zur Verfügung zu stellen, die die Nachteile der Multimetalloxidmassen des Standes der Technik nicht aufweisen. Demgemäß wurden die eingangs definierten Massen I gefunden.

Ähnliche Multimetalloxidmassen mit mehrphasigem Aufbau beschreiben die EP-A 669 164 und die EP-A 668 103.

Vorteilhafte Massen I sind solche, in denen $X^1$ Phosphor ist. Ferner sind solche Massen I günstig, in denen $X^2$ Vanadium ist. Auch ist es von Vorteil, wenn 3 bis 30 mol-% von $X^3$ Kalium, Rubidium, Caesium und/oder Ammonium sind. Als bevorzugter Wasserstoffersatz wird Caesium verwendet. $X^4$ ist vorzugsweise Antimon und $X^5$ ist mit Vorteil Rhodium. Als $X^6$ kommt in vorteilhafter Weise Molybdän zum Einsatz und als $X^7$ werden vorzugsweise Caesium und/oder Ammonium ($NH_4$), insbesondere Ammonium angewendet.

Ferner ist es von Vorteil, wenn wenigstens einer der beiden Anteile $[A]_p$, $[B]_q$ der erfindungsgemäßen Multimetalloxidmassen in den letzteren in Form dreidimensional ausgedehnter Bereiche der chemischen Zusammensetzung A bzw. B enthalten ist, deren Größtdurchmesser $d_A$ bzw. $d_B$ (längste durch den Schwerpunkt des Bereichs gehende Verbindungsstecke zweier auf der Oberfläche (Grenzfläche) des Bereichs befindlicher Punkte) > 0 bis 200 µm, vorzugsweise 1 bis 200 µm, beträgt. Ein besonders günstiger Größtdurchmesserbereich ist 1 bis 50 µm und ganz besonders vorteilhaft ist der Bereich 1 bis 30 µm. Selbstverständlich können die Größtdurchmesser aber auch 50 bis 150 µm oder 75 bis 125 µm betragen (die experimentelle Ermittlung der Größtdurchmesser gestattet z.B. die Methode der energiedispersiven Röntgenanalyse (EXDS), z.B. an einer Elektronenstrahl-Mikrosonde JEOL JCXA/733).

Vorzugsweise liegt sowohl der Anteil $[A]_p$ (die Co-Phase) als auch der Anteil $[B]_q$ (die Schlüsselphase) in den erfindungsgemäßen Multimetalloxidmassen im wesentlichen in kristalliner Form vor. D.h. in der Regel bestehen sowohl die Bereiche A als auch die Bereiche B im wesentlichen aus kleinen Kristalliten, deren Größtausdehnung in typischer Weise 0,1 bis 1 µm beträgt.

Von besonders günstiger Beschaffenheit sind solche Multimetalloxidmassen, deren Bereiche A im wesentlichen aus Kristalliten bestehen, deren Strukturtyp demjenigen des Ammoniumsalzes der Molybdatophosphorsäure $((NH_4)_3 PO_4 (MoO_3)_{12} \cdot 4H_2O)$ entspricht. Das Vorhandensein dieses Kristallstrukturtyps läßt sich z.B. dadurch nachweisen, daß die Röntgenbeugungsaufnahme der erfindungsgemäßen Multimetalloxidmasse das Beugungsmuster des Ammoniumsalzes der Molybdatophosphorsäure (Fingerabdruck) enthält, wobei je nach Elementzusammensetzung geringe Unterschiede bezüglich der Intensität und der Lage der Beugungslinien möglich sind. Der Röntgenbeugungsfingeraodruck des Ammoniumsalzes der Molybdatophosphorsäure ist z.B. veröffentlicht in Karteikarte 9-412 der JCPS-ICDD Kartei (1991), die dem Fachmann bekannt und allgemein zugänglich ist. Eine andere Quelle ist Natl. Bur. Stand. (U.S.), Circ. 539, 8 10 (1959). Umfaßt der Anteil $[A]_p$ das Element Antimon, wird dieses im Unterschied zu den übrigen möglichen Konstituenten dieses Anteils nicht in die den Strukturtyp des Ammoniumsalzes der Molybdatophosphorsäure aufweisenden Kristallite eingebaut und befindet sich an der Oberfläche dieser Kristallite bzw. in deren Zwischenräumen. Von Vorteil ist es, wenn sich das Antimon zu 85 bis 95 % seines Gewichtes als Senarmontit (eine kristalline Erscheinungsform des Antimontrioxids) in Räumen zwischen aus im wesentlichen den übrigen Elementen gebildeten Kristalliten des Strukturtyps des Ammoniumsalzes der Molybdatophosphorsäure befindet, während 5 bis 15 % seines Gewichtes in der Oberfläche solcher Kristallite in amorpher Lösung vorliegen (die Herstellung von Senarmontit enthaltenden Multimetalloxidmassen lehrt die ältere Anmeldung DE-A 43 29 907 (O.Z. 0050/44276).

Ferner sind solche Multimetalloxidmassen bevorzugt, deren Bereiche B im wesentlichen aus Kristalliten bestehen, die das Beugungsmuster (den Strukturtyp) wenigstens einer der nachfolgenden Substanzen aufweisen:

$(NH_4)_2Cu(MoO_4)_2$ (Karteikarte 40-1490 der JCPDS-ICCD-Kartei (1991)),

$(NH_4)_2Cu(MoO_4)_2(NH_3)_2$ (Garin, L. & Costamaga J., Powder Diffraction Vol. 4, No. 4 (1989) S. 233) ,

$NaCu(OH)(MoO_4)$ (Clearfield et al., Inorg. Chem. 25 (1986) S. 3782),

$CuMoO_4$ (Karteikarte 22-242 der JCPDS-ICDD Kartei (1991)),

$Cu_2MoO_5$ (Karteikarte 22-607 der JCPDS-ICDD Kartei (1989)),

$Cu_2Mo_3O_{10}$ (Karteikarte 35-16 der JCPDS-ICDD Kartei (1991)),

$Cu_3Mo_2O_9$ (Karteikarte 24-55 und 34-637 der JCPDS-ICDD Kartei (1991)) ,

$Cu_3(MoO_4)_2(OH)_2$ (Lindgrenit, Karteikarte 36-405 der JCPDS-ICDD Kartei (1991)),

$Cu_4Mo_5O_{17}$ (Karteikarte 39-181 der JCPDS-ICDD Kartei (1991)),

$Cu_{4-x}Mo_3O_{12}$ mit x = 0 bis 0,15 (Karteikarten 24-56, 26-547 und 35-18 der JCPDS-ICDD Kartei (1989)),

$Cu_4Mo_6O_{20}$ (Clearfield et al., Inorg. Chem. Vol. 25 (1986) S. 3782)),

$Cu_6Mo_4O_{15}$ (Karteikarten 38-1380 und 35-17 der JCPDS-ICDD Kartei (1991)) ,

$Cu_6Mo_5O_{18}$ (Karteikarte 40-865 der JCPDS-ICDD Kartei 1991));

Häufig liegen Gemische verschiedener Strukturtypen vor.

Die erfindungsgemäßen Massen I sind in einfacher Weise z.B. dadurch erhältlich, daß man die Oxometallate

$$X \overset{6}{_{12}} Cu_g X \overset{7}{_h} O_y \qquad\qquad (B)$$

zunächst in feinteiliger Form getrennt vorbildet (Ausgangsmasse 1). Die Oxometallate B können dadurch hergestellt werden, daß man von geeigneten Quellen ihrer elementaren Konstituenten ein möglichst inniges, vorzugsweise feinteiliges, Trockengemisch herstellt und dieses bei Temperaturen von 200 bis 650°C, vorzugsweise 300 bis 500°C, mehrere Stunden calciniert(prinzipiell kann auch ein nicht calciniertes, durch Sprühtrocknung einer wäßrigen Lösung oder Suspension erhaltenes Trockengemisch als Ausgangsmasse 1 eingesetzt werden, die calcinierte Variante ist jedoch bevorzugt). Die Calcinierung kann unter Inertgas, unter einem Gemisch aus Inertgas und Sauerstoff (z.B. Luft), aber auch unter einem Gemisch aus $O_2$ und reduzierend wirkenden Gasen wie Kohlenwasserstoffen, Methacrolein oder Ammoniak erfolgen. Im letzteren Fall muß lediglich beachtet werden, daß der Konstituent Kupfer nicht bis zum Element reduziert wird. In der Regel nimmt die erforderliche Calcinationsdauer mit zunehmender Calcinierungstemperatur ab. Wesentlich ist, daß es sich bei den Elementquellen entweder bereits um Oxide handelt, oder um solche Verbindungen, die durch Erhitzen, gegebenenfalls in Anwesenheit von Sauerstoff, in Oxide überführbar sind. Neben den Oxiden kommen daher als Ausgangsverbindungen vor allem Halogenide, Nitrate, Formiate, Oxalate, Acetate, Carbonate oder Hydroxide in Betracht. Besonders geeignete Ausgangsverbindungen sind z.B. Ammoniumheptamolybdat, Ammoniumnitrat, Kupfer(II)nitrat, -sulfat und Alkalinitrate.

Das innige Vermischen der Ausgangsverbindungen kann in trockener oder in nasser Form erfolgen. Erfolgt es in trockener Form, werden die Ausgangsverbindungen zweckmäßigerweise als feinteilige Pulver eingesetzt. Vorzugsweise erfolgt das innige Vermischen jedoch in nasser Form. Üblicherweise werden die Ausgangsverbindungen dabei in Form einer wäßrigen Lösung und/oder Suspension miteinander vermischt. Nach Abschluß des Mischvorgangs wird die fluide Masse getrocknet und nach Trocknung calciniert. Vorzugsweise erfolgt die Trocknung durch Sprühtrocknung (Eingangstemperatur: 250 bis 600°C, Ausgangstemperatur: 80 bis 130°C). Nach erfolgter Calcinierung kann nochmals zerkleinert (z.B. durch Naß- oder Trockenmahlen, z.B. in der Kugelmühle oder durch Strahlmahlen) und aus dem dabei erhältlichen, in der Regel aus im wesentlichen kugelförmigen Partikeln bestehenden, Pulver die Kornklasse mit einem im für die Masse I gewünschten Größtdurchmesserbereich liegenden Korngrößtdurchmesser (in der Regel > 0 bis 200 μm, üblicherweise 1 bis 200 μm, vorzugsweise 1 bis 50 μm und besonders bevorzugt 1 bis 30 μm) durch in an sich bekannter Weise durchzuführendes Klassieren (z.B. Naß- oder Trockensiebung) abgetrennt werden. Die einzelne Pulverpartikel besteht in der Regel aus zahlreichen Kristalliten, deren Größtausdehnung in typischer Weise 0,1 bis 1 μm beträgt.

In entsprechender Weise wird aus in gleicher Weise geeigneten Quellen der elementaren Konstituenten der Oxometallate A

$$Mo_{12} \, X_a^1 \, X_b^2 \, X_c^3 \, X_d^4 \, S_e \, X_f^5 \, O_x \qquad\qquad (A)$$

ein feinteiliges inniges Trockengemisch erzeugt, das in der Regel jedoch nicht vorcalciniert wird (Ausgangsmasse 2). Wird die Ausgangsmasse 2 vorcalciniert eingesetzt, so erfolgt die Calcinierung zweckmäßig bei 250 bis 450°C (Inertgas, Luft). Besonders geeignete Ausgangsverbindungen sind:

| | |
|---|---|
| Mo: | Ammoniumheptamolybdat, |
| V: | Ammoniummetavanadat, |
| P: | 70 bis 100 gew.-%ige, vorzugsweise 76 bis 85 gew.-%ige Phosphorsäure, |
| Sb: | Senarmontit, |
| S: | Ammoniumsulfat, |
| Re: | Rheniumpentoxid oder Ammoniumperrhenat, |
| B: | Borsäure, |
| As: | Arsentrioxid, |
| Si: | Wasserglas, |
| Nb: | Ammoniumnioboxalat oder Ammoniumniobat, |
| Alkalimetalle: | Alkalimetallnitrate, |
| $NH_4$: | Ammoniumsulfat, -nitrat oder -carbonat, |
| Bi: | Wismutnitrat. |

Die Ausgangsmasse 1 und die Ausgangsmasse 2 werden anschließend im gewünschten Mengenverhältnis naß oder trocken miteinander vermischt (vorzugsweise trocken), das Gemisch geformt und dann bei Temperaturen von 250 bis 450°C mehrere Stunden calciniert. Die Calcinierung kann unter Inertgas, aber auch unter einem Gemisch aus Inertgas und Sauerstoff wie z.B. Luft erfolgen.

Das Formen des Gemisches aus der Ausgangsmasse 1 und der Ausgangsmasse 2 kann z.B. durch verdichten (z.B. Tablettieren, Extrudieren oder Strangpressen), gegebenenfalls unter Zusatz an sich üblicher Hilfsmittel, z.B. Graphit oder Stearinsäure als Gleitmittel, erfolgen. Im Falle von Vollkatalysatoren erfolgt das Verdichten unmittelbar zur gewünschten Katalysatorgeometrie, wobei als solche Hohlzylinder mit einem Außendurchmesser und einer Länge von 2 bis 10 mm und einer Wandstärke von 1 bis 3 mm bevorzugt werden. Ganz generell kann das Gemisch aus der Ausgangsmasse 1 und der Ausgangsmasse 2 sowohl vor als auch nach dem Calcinieren geformt werden. Dies kann z.B. auch so erfolgen, daß man das Gemisch nach dem Calcinieren zerkleinert und inerte Träger zur Herstellung von Schalenkatalysatoren aufbringt. Das Aufbringen kann aber auch bereits vor der abschließenden Calcinierung erfolgen. In diesem Fall erfolgt das Aufbringen vorzugsweise gemäß der EP-B 293 859. Selbstverständlich können die erfindungsgemäßen Massen I auch in Pulverform eingesetzt werden.

Die erfindungsgemäßen Massen I eignen sich insbesondere als Katalysatoren mit erhöhter Selektivität bei vorgegebenem Umsatz, erhöhter Aktivität, verlängerter Standzeit und verbesserter Reproduzierbarkeit in der Herstellung zur gasphasenkatalytisch oxidativen Herstellung von Methacrylsäure aus Methacrolein.

Die katalytische Gasphasenoxidation von Methacrolein zu Methacrylsäure unter Anwendung der erfindungsgemäßen Katalysatoren kann in an sich bekannter, z.B. in der DE-A 40 22 212 dargestellter, Weise erfolgen.

Desgleichen gilt für die Abtrennung der Methacrylsäure aus dem Produktgasstrom. Das Oxidationsmittel Sauerstoff kann z.B. in Form von Luft, aber auch in reiner Form eingesetzt werden.

Aufgrund der hohen Reaktionswärme werden die Reaktionspartner vorzugsweise mit Inertgas wie $N_2$, $CO_2$, gesättigten Kohlenwasserstoffen und/oder mit Wasserdampf verdünnt. Vorzugsweise wird bei einem Methacrolein : Sauerstoff : Wasserdampf : Inertgas Verhältnis von 1:(1bis3) : (2bis20) : (3bis30), besonders bevorzugt von 1:(1bis3) : (3bis10) : (7bis18) gearbeitet. Das eingesetzte Methacrolein kann auf verschiedene Weise erhalten worden sein, z.B. durch Gasphasenoxidation von Isobuten, tert.-Butanol oder dem Methylether von tert.-Butanol. Mit Vorteil wird Methacrolein eingesetzt, das durch Kondensation von Propanol mit Formaldehyd in Gegenwart von sekundären Aminen und Säuren in flüssiger Phase gemäß den in der DE-PS 875 114 oder in der DE-AS 28 55 514 beschriebenen Verfahren erhältlich ist. Die Gasphasenoxidation kann sowohl in Wirbelschichtreaktoren als auch in Festbettreaktoren ausgeführt werden. Vorzugsweise wird sie in Rohrbündelreaktoren durchgeführt, in deren Röhren die Katalysatormasse, vorzugsweise in Gestalt zylindrisch geformter Partikel, fest angeordnet ist. Die Reaktionstemperatur beträgt in der Regel 250 bis 350°C, der Reaktionsdruck liegt üblicherweise im Bereich von 1 bis 3 bar und die Gesamtraumbelastung beträgt vorzugsweise 800 bis 1800 Nl/l/h. Unter diesen Bedingungen liegt der Methacroleinumsatz bei einfachem Reaktordurchgang üblicherweise bei 60 bis 90 mol-%. Interessanterweise behalten die erfindungsgemäßen Massen ihre Eigenschaften über eine erhöhte Betriebsdauer im wesentlichen unverändert bei.

Bei dem beschriebenen Verfahren wird üblicherweise jedoch nicht reine Methacrylsäure, sondern ein Produktgemisch erhalten, von welchem die Methacrylsäure nachfolgend abgetrennt werden muß. Dies kann in an sich bekannter Weise erfolgen, z.B. indem man die Reaktionsgase nach indirekter und/oder direkter Kühlung bei Temperaturen von 40 bis 80°C mit Wasser wäscht, wobei eine wäßrige Methacrylsäurelösung erhalten wird, aus der die Methacrylsäure üblicherweise durch Extraktion mit einem organischem Lösungsmittel entfernt und von selbigem durch Destillation abgetrennt wird.

Neben der gasphasenkatalytischen Oxidation von Methacrolein zu Methacrylsäure vermögen die erfindungsgemäßen Massen I aber auch die gasphasenkatalytische Oxidation- und Ammonoxidation von anderen gesättigten, ungesättigten und aromatischen Kohlenwasserstoffen, Alkoholen, Aldehyden und Aminen zu katalysieren.

Genannt sei insbesondere die gasphasenkatalytische Oxidation von anderen 3 bis 6 C-Atome aufweisenden Alkanen, Alkanolen, Alkanalen, Alkenen und Alkenolen (z.B. Propylen, Acrolein, tert.-Butanol, Methylether des tert.-Butanol, iso-Buten, iso-Butan oder iso-Butyraldehyd zu olefinisch ungesättigten Aldehyden und/oder Carbonsäuren, sowie den entsprechenden Nitrilen (Ammonoxidation, vor allem von Propen zu Acrylnitril und von iso-Buten bzw. tert.-Butanol zu Methacrylnitril). Besonders hervorgehoben sei die Herstellung von Acrylsäure, Acrolein und Methacrolein, sowie die Oxidation von n-Butan zu Maleinsäureanhydrid und diejenige von Butadien zu Furan. Sie eignen sich aber auch zur oxidativen Dehydrierung organischer Verbindungen.

Umsatz, Selektivität und Verweilzeit sind in dieser Schrift, falls nichts anderes erwähnt wird, wie folgt definiert:

$$\text{Umsatz U an Methacrolein (\%)} = \frac{\text{Molzahl umgesetztes Methacrolein}}{\text{Molzahl eingesetztes Methacrolein}} \times 100$$

$$\text{Selektivität S der Methacrylsäurebildung} = \frac{\text{Molzahl Methacrolein umgesetzt zu Methacrylsäure}}{\text{Molzahl Methacrolein insgesamt umgesetzt}} \times 100$$

$$\text{Verweilzeit (sec)} = \frac{\text{mit Katalysator gefülltes Leervolumen des Reaktors (l)}}{\text{durchgesetzte Synthesegasmenge (Nl/h)}} \times 3600$$

Beispiel

a) Herstellung von erfindungsgemäßen Multimetalloxidmassen M und Multimetalloxidmassen MV zum Vergleich (den Gehalt an Wasserstoff, Ammonium und Sauerstoff der resultierenden Massen legt das jeweilige Herstellungsverfahren fest; die Werte wurden nicht regelmäßig ermittelt und sind daher in den angegebenen Stöchiometrien nicht regelmäßig enthalten)

MV1 : Example 3 der EP-A 446 644 wurde nachgearbeitet. Als Antimonquelle wurde dabei reiner feinteiliger Senarmontit eines zahlenmittleren Partikeldurchmessers von 2,4 µm eingesetzt. Als Katalysatorgeometrie wurden Hohlzylinder der Ausmaße 7 mm (Höhe) x 7 mm (Außendurchmesser) x 3 mm (Innendurchmesser) gewählt. Dem resultierenden Katalysator liegt nachfolgende Stöchiometrie zugrunde:

$$P_{1,5} Mo_{12} V_{0,5} Cu_{0,1} K_1 Bi_{0,5} Sb_{0,3} B_{0,5}$$

MV2 : Example 6 der EP-A 446 644 wurde nachgearbeitet (Katalysatorgeometrie: 7 mm x 7 mm x 3 mm Hohlzylinder). Als Antimonquelle wurde reiner feinteiliger Senarmontit eines zahlenmittleren Partikeldurchmessers von 1,5 µm eingesetzt (Katalysatorgeometrie: 7 mm x 7 mm x 3 mm). Resultierende Katalysatorstöchiometrie:

$$P_{1,5} Mo_{12} V_{0,8} Cu_{0,1} K_{0,7} Cs_{0,4} Bi_{0,3} Sb_{0,3} Ge_{0,2} As_{0,2}$$

M1 : Ausgangsmasse 2: In 1150 g Wasser wurden nacheinander 980 g Ammoniumheptamolybdat, 27,6 g Ammoniummetavanadat und 47,7 g Kaliumnitrat eingerührt. Die Temperatur der resultierenden wäßrigen Mischung wurde auf 30 bis 35°C eingestellt. Dann wurden 90,66 g 76 gew.-%ige Phosphorsäure zugegeben und die Temperatur der Mischung auf 45 bis 48°C erhöht. Anschließend wurde eine Lösung von 49,3 g Wismutnitrat in verdünnter Salpetersäure (hergestellt durch Mischen von 300 g Wasser und 30 g 50 gew.-%iger Salpetersäure) zugesetzt und das wäßrige Gemisch innerhalb von 30 bis 35 min auf 95°C erhitzt. Danach wurden nacheinander 20,9 g feinteiliges Antimontrioxid (reiner Senarmontit) eines zahlenmittleren Partikeldurchmessers von 2,4 µm und 14,59 g Borsäure hinzugegeben und die Mischung 30 min bei 95°C gerührt. Anschließend wurde die Mischung bei einer Austrittstemperatur von 130°C sprühgetrocknet. Der resultierenden Ausgangsmasse liegt folgende Stöchiometrie zugrunde:

$P_{1,52} Mo_{12} V_{0,51} K_{1,02} Bi_{0,51} Sb_{0,31} B_{0,51}$ Ausgangsmasse 1: Nach L. Garin, J. Costamaga in Powder Diffraction Vol. 4, No. 4 (1989) S. 233 wurde $(NH_4)_2 Cu (MoO_4)_2$ in feinteiliger Form hergestellt.

Die Ausgangsmasse 2 und die Ausgangsmasse 1 wurden im molaren Verhältnis 0,98/0,1 trocken gemischt. Nach Zusatz von 3 Gew.-% Graphit wurde die Trockenmasse zu Hohlzylindern (7 mm x 7 mm x 3 mm) tablettiert und 5 h lang bei 380°C im Luftstrom calciniert.

Die Stöchiometrie der resultierenden Masse M1 entspricht derjenigen von MV1.

M2 : Ausgangsmasse 2: In 1500 g Wasser wurden nacheinander 980 g Ammoniumheptamolybdat, 43,83 g Ammoniummetavanadat und 33,2 g Kaliumnitrat eingerührt. Die Temperatur der resultierenden wäßrigen Mischung wurde auf 30 bis 35°C eingestellt. Anschließend wurden 90,66 g 76 gew.-%ige Phosphorsäure zugesetzt und die Temperatur auf 40 bis 45°C erhöht. Dann wurde eine Lösung von 33,41 g Wismutoxid in verdünnter Salpetersäure (hergestellt aus 300 g Wasser und 30 g 60 gew.-%iger Salpetersäure) und anschließend ein Gemisch aus 9,15 g Arsenoxid, 9,68 g Germaniumdioxid und 50 g 15 %ige wäßrige Ammoniaklösung eingerührt. Das resultierende Gemisch wurde innerhalb von 30 bis 35 min gleichmäßig auf 95°C erhitzt. Nach Erreichen von 70°C wurde auf einmal eine Lösung von 36,97 g Caesiumnitrat in 80 g Wasser und danach 20,9 g feinteiliges Antimontrioxid (reiner Senarmontit) mit einem zahlenmittleren Partikeldurchmesser von 1,5 μm zugegeben. Das resultierende Gemisch wurde 30 min bei 95°C gerührt und anschließend bei einer Austrittstemperatur von 110°C sprühgetrocknet.

Der resultierenden Ausgangsmasse 2 liegt folgende Stöchiometrie zugrunde:

$P_{1,52} Mo_{12} V_{0,81} K_{0,71} Cs_{0,41} Bi_{0,31} Sb_{0,31} Ge_{0,2} As_{0,2}$

Ausgangsmasse 1: Wie bei M1. Zusätzlich wurde auf einen Partikeldurchmesser < 50 μm gemahlen. Das Vermischen der Ausgangsmasse 2 und der Ausgangsmasse 1 und die Fertigstellung des Katalysators erfolgte wie bei M1.

Die Stöchiometrie der resultierenden Masse M2 entspricht derjenigen von MV2.

M3 : Ausgangsmasse 2: In 1200 g Wasser wurden nacheinander 1000 g Ammoniumheptamolybdat, 55,21 g Ammoniummetavanadat und 128,8 g Caesiumnitrat eingerührt. Die Temperatur der resultierenden wäßrigen Mischung wurde anschließend auf 40 bis 45°C eingestellt. Dann wurden nacheinander 91,29 g 76 gew.-%ige Phosphorsäure und 2,49 g Ammoniumsulfat eingerührt. Danach wurde die Temperatur der Mischung auf 45 bis 48°C erhöht. Daraufhin wurden 51,6 g feinteiliges Antimontrioxid (reiner Senarmontit) mit einem zahlenmittleren Partikeldurchmesser von 3,2 μm hinzugefügt, das Gemisch auf 95°C erwärmt und eine Stunde lang bei dieser Temperatur gerührt. Abschließend wurde bei einer Austrittstemperatur von 110°C sprühgetrocknet.

Der resultierenden Ausgangsmasse 2 liegt folgende Stöchiometrie zugrunde:

$P_{1,5} Mo_{12} V_1 Cs_{1,4} S_{0,04} Sb_{0,75}$

Ausgangsmasse 1: Wie bei M1.

Das trockene Vermischen der Ausgangsmasse 2 und der Ausgangsmasse 1 erfolgte im molaren Verhältnis 1/0,5 und die Fertigstellung des Katalysators erfolgte wie bei M1.

M4 : Wie M3, das molare Mischungsverhältnis der Ausgangsmasse 2 zur Ausgangsmasse 1 betrug jedoch 1/1.

M5 : Ausgangsmasse 2: Wie bei M3. Ausgangsmasse 1: Nach Clearfield et al. in Inorg. Chem. 25 (1986) S. 3782 wurde feinteiliges $Cu_3 (MoO_4)_2 (OH)_2$ hergestellt.

Das trockene Vermischen der Ausgangsmasse 2 und der Ausgangsmasse 1 erfolgte im molaren Verhältnis 1/0,25 und die Fertigstellung des Katalysators erfolgte wie bei M1.

M6 : Wie M5, das molare Mischungsverhältnis der Ausgangsmasse 2 und der Ausgangsmasse 1 betrug jedoch 1/1.

M7 : Ausgangsmasse 2: In 1000 g Wasser wurden nacheinander 1000 g Ammoniumheptamolybdat, 55,21 g Ammoniummetavanadat und 60 % einer Lösung von 110,4 g Caesiumnitrat in 250 g Wasser eingerührt. Die Temperatur der Mischung wurde auf 37 bis 42°C eingestellt. Danach wurden nacheinander 115,64 g 76 gew.-%ige Phosphorsäure und 2,49 g Ammoniumsulfat eingearbeitet und die Mischungstemperatur auf 40 bis 45°C erhöht. Dann wurden 68,79 g feinteiliges Antimontrioxid (25 % Valentinit, 75 % Senarmontit) mit einem zahlenmittleren Partikeldurchmesser von 3,2 μm eingearbeitet und die Mischung auf 95°C erwärmt. Während der Aufheizphase wurden die verbliebenen 40 % der wäßrigen Caesiumnitratlösung, aufgeteilt in drei gleiche Portionen, beim Erreichen der Temperaturen von 80, 90 und 95°C jeweils auf einmal zugegeben und anschließend bei einer Austrittstemperatur von 110°C sprühgetrocknet.

Der resultierende Ausgangsmasse 2 liegt folgende Stöchiometrie zugrunde:

$$P_{1,9} \, Mo_{12} \, V_1 \, Cs_{1,2} \, S_{0,04} \, Sb_1$$

Ausgangsmasse 1: In Abwandlung der von L. Garin, J. Costamaga in Powder Diffraction Vol. 4, No. 4 (1989) S. 233 gegebenen Herstellvorschrift (der Kupfersulfatlösung wurde in entsprechender Menge Caesiumnitrat zugesetzt) wurde feinteiliges

$$Cs_{1,67} \, (NH_4)_{0,33} \, Cu \, (MoO_4)_2$$

hergestellt.

Die Ausgangsmasse 2 und die Ausgangsmasse 1 wurden trocken im molaren Verhältnis 1/0,5 gemischt. Die Fertigstellung des Katalysators erfolgte wie bei M1.

M8 : Ausgangsmasse 2: Wie bei M3.
Ausgangsmasse 1: Feinteiliges $CuMoO_4$, hergestellt gemäß K. Nassau, J.W. Shiever in J. Am. Ceram. Soc. 52 (1969) 36.
Die Ausgangsmasse 2 und die Ausgangsmasse 1 wurden trocken im molaren Verhältnis 1/1,5 gemischt. Die Fertigstellung des Katalysators erfolgte wie bei M1.

M9 : Ausgangsmasse 2: Wie bei M3.
Ausgangsmasse 1: Feinteiliges $Cu_4 \, Mo_5 \, O_{17}$, hergestellt gemäß E.M. McCarron, J.C. Calabrese in J. Solid State Chem. 65 (1986) 215.
Die Ausgangsmasse 2 und die Ausgangsmasse 1 wurden trocken im molaren Verhältnis 1/0,17 gemischt. Die Fertigstellung des Katalysators erfolgte wie bei M1.

M10 : Ausgangsmasse 2: Wie bei M3.
Ausgangsmasse 1: Feinteiliges $Cu_6 \, Mo_5 \, O_{18}$, hergestellt gemäß E.M. McCarron, J.C. Calabrese in J. Solid State Chem. 62 (1986) 65.
Die Ausgangsmasse 2 und die Ausgangsmasse 1 wurden trocken im molaren Verhältnis 1/0,33 gemischt. Die Fertigstellung des Katalysators erfolgte wie bei M1.

M11 : Ausgangsmasse 2: Wie bei M3.
Ausgangsmasse 1: Feinteiliges $Cu_6 \, Mo_5 \, O_{18}$, hergestellt gemäß E.M. McCarron, J.C. Calabrese in J. Solid State Chem. 62 (1986) 65, gemischt mit feinteiligem $Cu_3 \, Mo_2 \, O_9$, hergestellt gemäß Katz et al. in Acta Cryst. B 27 (1971) 2071, im molaren Verhältnis 1/1.
Die Ausgangsmasse 2 und die Ausgangsmasse 1 wurden trocken im molaren Verhältnis 1/0,33 gemischt. Die Fertigstellung des Katalysators erfolgte wie bei M1.

M12 : Ausgangsmasse 2: Wie bei M3.
Ausgangsmasse 1: Feinteiliges $KCu(OH) \, (MoO_4)$, hergestellt gemäß Clearfield et al. in Inorg. Chem 25 (1986) 3782, wobei Na durch K ersetzt wurde.
Die Ausgangsmasse 2 und die Ausgangsmasse 1 wurden trocken im molaren Verhältnis 1/0,33 gemischt. Die Fertigstellung des Katalysators erfolgte wie bei M1.

M13 : Ausgangsmasse 2: Wie bei M3.
Ausgangsmasse 1: Feinteiliges $Cu_2 \, Mo_3 \, O_{10}$, hergestellt gemäß T. Machej, J. Ziolkowski in J. Solid State Chem. 31 (1980) 136.
Die Ausgangsmasse 2 und die Ausgangsmasse 1 wurden trocken im molaren Verhältnis 1/0,5 gemischt. Die Fertigstellung des Katalysators erfolgte wie bei M1.

M14 : Ausgangsmasse 2: Wie bei M3.
Ausgangsmasse 1: Feinteiliges $Cu_{3,85} \, Mo_3 \, O_{12}$, hergestellt gemäß L. Katz in Acta Cryst. B27 (1971) 2071.
Die Ausgangsmasse 2 und die Ausgangsmasse 1 wurden trocken im molaren Verhältnis 1/0,5 gemischt. Die Fertigstellung des Katalysators erfolgte wie bei M1.

M15 : Wie M3, die Ausgangsmasse 2 und die Ausgangsmasse 1 wurden jedoch unter Zusatz von Wasser verknetet und zu 6 mm x 6 mm Vollzylinder extrudiert, getrocknet und gemäß M1 calciniert.

M16 : Ausgangsmasse 2: In 1000 g Wasser wurden nacheinander 1000 g Ammoniumheptamolybdat, 55,21 g Ammoniummetavanadat und 60 % einer Lösung von 128,8 g Caesiumnitrat in 250 g Wasser eingerührt. Die Temperatur der resultierenden wäßrigen Mischung wurde auf 37 bis 42°C eingestellt. Anschließend wurden 91,29 g 76 gew.-%ige Phosphorsäure und 2,49 g Ammoniumsulfat eingearbeitet. Dann wurde die Temperatur des wäßrigen Gemischs auf 40 bis 45°C eingestellt. Daraufhin wurden 51,6 g feinteiliges Antimontrioxid (25 % Valentinit, 75 % Senarmontit) eines zahlenmittleren Partikeldurchmessers von 3,2 µm eingearbeitet und die Mischung auf 95°C erwärmt. Während der Aufheizphase wurden die verbliebenen 40 % der wäßrigen Caesiumnitratlösung, aufgeteilt in drei gleiche Portionen, beim Erreichen der Temperaturen von 80, 90 und 95°C jeweils auf einmal zugegeben. Das Gemisch wurde anschließend noch 30 min bei 95°C gerührt. Kurz vor der Sprühtrocknung wurde die feinteilige Ausgangsmasse 1 zugesetzt und anschließend bei einer Austrittstemperatur von 110°C sprühgetrocknet.
Ausgangsmasse 1: Wie bei M3.
Das molare Mischungsverhältnis von Ausgangsmasse 2 zu Ausgangsmasse 1 betrug 1/0,5. Das Sprühpulver wurde entsprechend M1 geformt und calciniert.

M17 : Ausgangsmasse 2: Wie bei M3.
Ausgangsmasse 1: Wie bei M12.
Die Ausgangsmasse 2 und die Ausgangsmasse 1 wurden im molaren Verhältnis 1/0,33 vermischt, unter Wasserzusatz geknetet, zu 6 mm x 6 mm Vollzylindern extrudiert, getrocknet und wie bei M1 calciniert.

M18 : Wie M16, das molare Mischungsverhältnis von Ausgangsmasse 2 zu Ausgangsmasse 1 betrug jedoch 1/0,33.

M19 : Ausgangsmasse 2: In 1000 g Wasser wurden nacheinander 1000 g Molybdäntrioxid, 52,65 g Vanadiumpentoxid und 112 g 76 gew.-%ige Phosphorsäure eingerührt. Das Gemisch wurde 12 h bei 95°C gerührt. Anschließend wurden geringe Mengen an Ungelöstem abfiltriert und die Lösung auf 50°C abgekühlt. Danach wurden zu der Lösung 154,2 g Tetrapropylammoniumbromid innerhalb von 2 h kontinuierlich zugegeben und nach beendeter Zugabe noch 1 h bei 50°C gerührt. Anschließend wurde bei einer Austrittstemperatur von 110°C sprühgetrocknet und danach bei 390°C 10 h lang calciniert.
Der resultierenden Ausgangsmasse 2 liegt nachfolgende Stöchiometrie zugrunde:
$$P_1 Mo_{12} V_1 H_4$$
Ausgangsmasse 1: Wie bei M8.
Die Ausgangsmasse 2 und die Ausgangsmasse 1 wurden trocken im molaren Verhältnis 1/0,5 gemischt. Nach Zusatz von 3 Gew.-% Graphit wurde zu 7 mm x 5 mm x 3 mm Hohlzylindern tablettiert und 5 h bei 380°C calciniert.

M20 : Wie M19, in der Ausgangsmasse 2 wurde jedoch die Hälfte des Phosphors durch Arsen (eingesetzt als Arsen(III)oxid) ersetzt.

M21 : Ausgangsmasse 2: In 5000 g Wasser wurden bei 60°C in der genannten Abfolge nacheinander 54,1 g Ammoniummetavanadat, 980 g Ammoniumheptamolybdat und 106,7 g 85 gew.-%ige wäßrige Phosphorsäure und 180,32 g Caesiumnitrat eingerührt. Das resultierende Gemisch wurde 1 h bei einer Temperatur von 60 bis 65°C gerührt und anschließend bei einer Austrittstemperatur von 120°C sprühgetrocknet.
Der resultierenden Ausgangsmasse 2 liegt folgende Stöchiometrie zugrunde:
$$P_2 Mo_{12} V_1 Cs_2$$
Ausgangsmasse 1: Nach K. Nassau & J.K. Shiever, J. Am. Chem. Soc. Vol. 52, No. 1 (1969), S. 36 wurde $Cu_2MoO_5$ in feinteiliger Form hergestellt (zahlenmittlerer Korngrößtdurchmesser = 32 µm).
Die Ausgangsmasse 2 und die Ausgangsmasse 1 wurden im molaren Verhältnis 1/0,33 trocken gemischt. Nach Zusatz von 3 Gew.-% Graphit wurde die Trockenmasse zu Hohlzylindern (7 mm x 7 mm x 3 mm) tablettiert und 5 h lang bei 380°C im Luftstrom calciniert.

MV3 : Ausgangsmasse 2: Wie bei M21.
Ausgangsmasse 1: Nach J. Aykan, J. Catal. Vol. 12 (1968) S. 281, wurde $Bi_2MoO_6$ in feinteiliger Form hergestellt (zahlenmittlerer Korngrößtdurchmesser = 32 µm).
Das Vermischen der Ausgangsmasse 2 und der Ausgangsmasse 1 und die Fertigstellung des Katalysators erfolgte wie bei M21.

M22 : Ausgangsmasse 2: In 1500 g Wasser wurden nacheinander 980 g Ammoniumheptamolybdat, 37,88 g Ammoniummetavanadat und 90,16 g Caesiumnitrat eingerührt. Die Temperatur der resultierenden Mischung wurde auf 30 bis 35°C eingestellt. Anschließend wurden 80 g 85 gew.-%ige wäßrige Phosphorsäure eingerührt. Danach wurde die Temperatur der Mischung auf 40 bis 45°C eingestellt und 67,22 g Antimontrioxid (75 % Senarmontit/25 % Valentinit) mit einem zahlenmittleren Partikeldurchmesser von 0,5 μm zugegeben. Die Mischung wurde 10 min bei 40 bis 45°C gerührt und anschließend bei einer Austrittstemperatur von 110°C sprühgetrocknet. Der resultierenden Ausgangsmasse 2 liegt folgende Stöchiometrie zugrunde:

$$P_{1,5}Mo_{12}V_{0,7}Cs_{1,0}Sb_{1,0}$$

Ausgangsmasse 1: Nach T. Machej & J. Ziolkowski, J. Solid State Chem. 31 (1980) S. 136 wurde $Cu_2Mo_3O_{10}$ in feinteiliger Form hergestellt (zahlenmittlerer Korngrößtdurchmesser = 25 μm).

Das Vermischen der Ausgangsmasse 2 und der Ausgangsmasse 1 und die Fertigstellung des Katalysators erfolgte wie bei M21.

MV4 : Wie M22, als Ausgangsmasse 1 wurde jedoch nach P.A. Batist, J.F. Bouwens und G.C.A. Schmit, J. Catal. Vol. 25 (1972) S. 1 hergestelltes feinteiliges $Bi_2(MoO_4)_3$ verwendet (zahlenmittlerer Korngrößtdurchmesser = 25 μm).

b) Verwendung der Multimetalloxidmassen aus a) als Katalysatoren für die Gasphasenoxidation von Methacrolein zu Methacrylsäure

Die Katalysatoren wurden in einem Rohrreaktor gefüllt (10 mm Innendurchmesser, 100 g Katalysatorschüttung, Salzbadtemperierung) und bei Reaktionstemperaturen im Bereich von 270 bis 300°C unter Anwendung einer Verweilzeit von 3,6 Sekunden mit einem gasförmigen Gemisch der Zusammensetzung

     5 Vol-% Methacrolein,
     10 Vol-% Sauerstoff,
     10 Vol-% Wasserdampf und
     75 Vol-% Stickstoff

beschickt. Die Salzbadtemperatur wurde im wesentlichen in allen Fällen so eingestellt, daß ein einheitlicher Methacroleinumsatz von ca. 89 % resultierte. Eine geringere Salzbadtemperatur weist eine erhöhte Katalysatoraktivität aus. Das aus dem Rohrreaktor strömende Produktgasgemisch wurde gaschromatographisch analysiert. Die Ergebnisse für die Selektivität der Methacrylsäurebildung in Anwendung der verschiedenen Katalysatoren zeigt die nachfolgende Tabelle.

Tabelle

| Katalysator | Reaktionstemperatur (°C) | U (%) | S (%) |
|---|---|---|---|
| MV1 | 290 | 88,7 | 89 |
| MV2 | 290 | 90,1 | 89 |
| M1 | 288 | 88,7 | 89,8 |
| M2 | 287 | 90,1 | 90,1 |
| M3 | 276 | 89,2 | 90,3 |
| M4 | 273 | 88,9 | 89,8 |
| M5 | 278 | 89,1 | 88,2 |
| M6 | 288 | 89,5 | 90,5 |
| M7 | 278 | 89 | 87,4 |
| M8 | 291 | 88,9 | 89 |
| M9 | 275 | 88,8 | 85 |

Tabelle (fortgesetzt)

| Katalysator | Reaktionstemperatur (°C) | U (%) | S (%) |
|---|---|---|---|
| M10 | 295 | 89 | 86,2 |
| M11 | 291 | 89,2 | 87,3 |
| M12 | 285 | 89,3 | 90 |
| M13 | 298 | 89,5 | 89,9 |
| M14 | 287 | 90 | 88,7 |
| M15 | 278 | 89,2 | 90 |
| M16 | 282 | 89,4 | 89,4 |
| M17 | 286 | 89,2 | 89,7 |
| M18 | 288 | 89,5 | 89,1 |
| M19 | 283 | 89,8 | 85,4 |
| M20 | 285 | 90 | 86,9 |
| M21 | 281 | 89,7 | 87,9 |
| MV3 | 295 | 89,7 | 82,1 |
| M22 | 293 | 88,2 | 89,2 |
| MV4 | 297 | 88,2 | 84,1 |

Abschließend sei festgestellt, daß die Röntgenbeugungsaufnahme aller Multimetalloxidmassen M1 bis M20 sowohl den Fingerabdruck des Ammoniumsalzes der Molybdatophosphorsäure ( $(NH_4)_3 PO_4 (MoO_3)_{12} . 4H_2O$ ) als auch einen Kupfermolybdatfingerabdruck aufwiesen.

**Patentansprüche**

1. Multimetalloxidmassen der Formel I

$$[A]_p [B]_q \qquad (I),$$

in der die Variablen folgende Bedeutung haben:

A $\quad Mo_{12} X_a^1 X_b^2 X_c^3 X_d^4 S_e X_f^5 O_x$

B $\quad X_{12}^6 Cu_g X_h^7 O_y$

$X^1$ $\quad$ Phosphor, Arsen, Bor, Germanium und/oder Silicium,

$X^2$ $\quad$ Vanadium, Niob und/oder Wolfram,

$X^3$ $\quad$ Wasserstoff, von dem bis zu 97 mol-% ersetzt sein können durch Kalium, Rubidium, Caesium und/oder Ammonium ($NH_4$),

$X^4$ $\quad$ Antimon und/oder Wismut,

$X^5$ $\quad$ Rhenium und/oder Rhodium,

$X^6$ $\quad$ Molybdän, Wolfram, Niob und/oder Tantal,

$X^7$ $\quad$ Lithium, Natrium, Kalium, Rubidium, Caesium und/oder Ammonium ($NH_4$),

a    1 bis 6,

b    0 bis 6,

c    3 bis 5,

d    0 bis 6,

e    0 bis 3,

f    0 bis 3,

g    4 bis 24,

h    0 bis 20,

x,y    Zahlen, die durch die Wertigkeit und Häufigkeit der von Sauerstoff verschiedenen Elemente in I bestimmt werden und

p,q    von Null verschiedene Zahlen, deren Verhältnis p/q 12:0,1 bis 12:48 beträgt,

die den Anteil $[A]_p$ in Form dreidimensional ausgedehnter, von ihrer lokalen Umgebung aufgrund ihrer von ihrer lokalen Umgebung verschiedenen chemischen Zusammensetzung abgegrenzter, Bereiche A der chemischen Zusammensetzung

A    $Mo_{12} X_a^1 X_b^2 X_c^3 X_d^4 S_e X_f^5 O_x$

und den Anteil $[B]_q$ in Form dreidimensional ausgedehnter, von ihrer lokalen Umgebung aufgrund ihrer von ihrer lokalen Umgebung verschiedenen chemischen Zusammensetzung abgegrenzter, Bereiche B der chemischen Zusammensetzung

B    $X_{12}^6 Cu_g X_h^7 O_y$

enthalten, wobei die Bereiche A, B relativ zueinander wie in einem Gemisch aus feinteiligem A und feinteiligem B verteilt sind.

2.    Multimetalloxidmassen nach Anspruch 1, in denen e 0,01 bis 1 ist.

3.    Multimetalloxidmassen nach Anspruchen 1 oder 2, in denen g 8 bis 15 beträgt.

4.    Multimetalloxidmassen nach den Ansprüchen 1 bis 3, in denen wenigstens einer der beiden Anteile $[A]_p$, $[B]_q$, in Form dreidimensional ausgedehnter Bereiche enthalten ist, deren Größtdurchmesser 1 bis 200 µm beträgt.

5.    Multimetalloxidmassen nach den Ansprüchen 1 bis 4, deren Röntgenbeugungsaufnahme den Fingerabdruck des Strukturtyps des Ammoniumsalzes der Molybdatophosphorsäure ( $(NH_4)_3 PO_4 (MoO_3)_{12} \cdot 4H_2O$) aufweist.

6.    Multimetalloxidmassen nach den Ansprüchen 1 bis 5, die das Element Antimon als Senarmontit enthalten.

7.    Multimetalloxidmassen nach den Ansprüchen 1 bis 6, deren Röntgenbeugungsaufnahme den Fingerabdruck eines Kupfermolybdats aufweist.

8.    Verfahren zur Herstellung von Multimetalloxidmassen gemäß Anspruch 1, durch gekennzeichnet, daß man ein Oxometallat B

B    $X_{12}^6 Cu_g X_h^7 O_y$

in feinteiliger Form vorbildet, und mit einem feinteiligen innigen Trockengemisch von Quellen der elementaren Konstituenten eines Oxometallats A

A      $Mo_{12} X_a^1 X_b^2 X_c^3 X_d^4 S_e X_f^5 O_x$

vermischt und das Gemisch bei einer Temperatur von 250 bis 450°C calciniert.

9. Verfahren zur gasphasenkatalytisch oxidativen Herstellung von Methacrylsäure aus Methacrolein, dadurch gekennzeichnet, daß als Katalysator ein Multimetalloxid gemäß der Ansprüche 1 bis 7 verwendet wird.

10. Verfahren nach Anspruch 9, dadurch gekennzeichnet, daß es sich bei dem verwendeten Katalysator um einen Vollkatalysator handelt, dessen Geometrie diejenige eines Hohlzylinders mit einem Außendurchmesser und einer Länge von 2 bis 10 mm und einer Wandstärke von 1 bis 3 mm ist.

**Claims**

1. A multimetal oxide composition of the formula I

$$[A]_p [B]_q \qquad\qquad (I),$$

where

A      is $Mo_{12} X_a^1 X_b^2 X_c^3 X_d^4 S_e X_f^5 O_x$

B      is $X_{12}^6 Cu_g X_h^7 O_y$

$X^1$      is phosphorus, arsenic, boron, germanium and/or silicon,

$X^2$      is vanadium, niobium and/or tungsten,

$X^3$      is hydrogen, of which up to 97 mol% may have been replaced by potassium, rubidium, cesium and/or ammonium ($NH_4$),

$X^4$      is antimony and/or bismuth,

$X^5$      is rhenium and/or rhodium,

$X^6$      is molybdenum, tungsten, niobium and/or tantalum,

$X^7$      is lithium, sodium, potassium, rubidium, cesium and/or ammonium ($NH_4$),

a      is from 1 to 6,

b      is from 0 to 6,

c      is from 3 to 5,

d      is from 0 to 6,

e      is from 0 to 3,

f      is from 0 to 3,

g      is from 4 to 24,

h      is from 0 to 20,

x and y      are numbers determined by the valency and frequency of the elements other than oxygen in I, and

p and q      are numbers other than zero whose ratio p/q is from 12:0.1 to 12:48,

which contains component $[A]_p$ in the form of three-dimensionally extended regions A of the chemical composition

$$A \qquad Mo_{12} X_a^1 X_b^2 X_c^3 X_d^4 S_e X_f^5 O_x$$

which are delimited from their local environment due to their chemical composition which is different from their local environment, and component $[B]_q$ in the form of three-dimensionally extended regions B of the chemical composition

$$B \qquad X_{12}^6 Cu_g X_h^7 O_y$$

which are delimited from their local environment due to their chemical composition which is different from their local environment, where the regions A and B are distributed relative to one another as in a mixture of finely divided A and finely divided B.

2. A multimetal oxide composition as claimed in claim 1, where e is from 0.01 to 1.

3. A multimetal oxide composition as claimed in claim 1 or 2, where g is from 8 to 15.

4. A multimetal oxide composition as claimed in any of claims 1 to 3, where at least one of the two components $[A]_p$ and $[B]_q$ is in the form of three-dimensionally extended regions whose maximum diameter is from 1 to 200 μm.

5. A multimetal oxide composition as claimed in any of claims 1 to 4, whose X-ray diffraction pattern contains the finger-print of the molybdatophosphoric acid ammonium salt structural type $((NH_4)_3PO_4(MoO_3)_{12} \cdot 4H_2O)$.

6. A multimetal oxide composition as claimed in any of claims 1 to 5, where the antimony is in the form of senarmontite.

7. A multimetal oxide composition as claimed in any of claims 1 to 6, whose X-ray diffraction pattern contains the finger-print of a copper molybdate.

8. A process for the preparation of a multimetal oxide composition as claimed in claim 1, which comprises pre-forming an oxometallate B

$$B \qquad X_{12}^6 Cu_g X_h^7 O_y$$

in the finely divided form, mixing this with a finely divided, intimate dry mix of sources of the elemental constituents of an oxometallate A

$$A \qquad Mo_{12} X_a^1 X_b^2 X_c^3 X_d^4 S_e X_f^5 O_x,$$

and calcining the mixture at from 250 to 450°C.

9. A process for the preparation of methacrylic acid from methacrolein by gas-phase catalytic oxidation, wherein the catalyst used is a multimetal oxide as claimed in any of claims 1 to 7.

10. A process as claimed in claim 9, wherein the catalyst used is an unsupported catalyst whose geometry is that of a hollow cylinder having an external diameter and a length of from 2 to 10 mm and a wall thickness of from 1 to 3 mm.

**Revendications**

1. Masses d'oxydes métalliques de formule I

$$[A]_p[B]_q \tag{I}$$

dans laquelle les variables ont les significations suivantes:

A        représente $Mo_{12} X_a^1 X_b^2 X_c^3 X_d^4 S_e X_f^5 O_x,$
B        représente $X_{12}^6 Cu_g X_h^7 O_y$

$X^1$ représente le phosphore, l'arsenic, le bore, le germanium et/ou le silicium,

$X^2$ représente le vanadium, le niobium et/ou le tungstène,

$X^3$ représente un atome d'hydrogène, dont jusqu'à 97 % en moles peuvent être remplacés par le potassium, le rubidium, le césium et/ou l'ammonium ($NH_4$),

$X^4$ représente l'antimoine et/ou le bismuth,

$X^5$ représente le rhénium et/ou le rhodium,

$X^6$ représente le molybdène, le tungstène, le niobium et/ou le tantale,

$X^7$ représente le lithium, le sodium, le potassium, le rubidium, le césium et/ou l'ammonium ($NH_4$),

a vaut de 1 à 6,

b vaut de 0 à 6,

c vaut de 3 à 5,

d vaut de 0 à 6,

e vaut de 0 à 3,

f vaut de 0 à 3,

g vaut de 4 à 24,

h vaut de 0 à 20,

x, y représentent des nombres qui sont déterminés par la valence et la fréquence des éléments autres que l'oxygène dans I, et

p, q sont des nombres différents de zéro, dont le rapport p/q va de 12:0,1 à 12:48,

qui contiennent la fraction $[A]_p$ sous forme de zones A à extension tridimensionnelle, délimitées de leur environnement local en raison de leur composition chimique différente de celle de leur environnement local, de composition chimique

A $\quad Mo_{12} X_a^1 X_b^2 X_c^3 X_d^4 S_e X_f^5 O_x$,

et la fraction $[B]_q$ sous forme de zones B à extension tridimensionnelle, délimitées de leur environnement local en raison de leur composition chimique différente de celle de leur environnement local, de composition chimique

B $\quad X_{12}^6 Cu_g X_h^7 O_y$

les zones A, B étant réparties les unes par rapport aux autres comme dans un mélange de A finement divisée et B finement divisée.

2. Masses d'oxydes multimétalliques selon la revendication 1, dans lesquelles e va de 0,01 à 1.

3. Masses d'oxydes multimétalliques selon la revendication 1 ou 2, dans lesquelles g va de 8 à 15.

4. Masses d'oxydes multimétalliques selon les revendications 1 à 3, dans lesquelles est contenue au moins l'une des deux fractions $[A]_p$, $[B]_q$ sous forme de zones à extension tridimensionnelle, dont le diamètre maximum va de 1 à 200 µm.

5. Masses d'oxydes multimétalliques selon les revendications 1 à 4, dont le diagramme de diffraction des rayons X présente les caractéristiques du type de structure du sel d'ammonium de l'acide molyodatophosphorique $[(NH_4)_3PO_4(MoO_3)_{12}.4H_2O]$.

6. Masses d'oxydes métalliques selon les revendications 1 à 5, qui contiennent l'élément antimoine sous forme de sénarmontite.

7. Masses d'oxydes métalliques selon les revendications 1 à 6, dont le diagramme de diffraction des rayons X présente les caractéristiques d'un molybdate de cuivre.

8. Procédé pour la préparation de masses d'oxydes métalliques selon la revendication 1, caractérisé en ce que l'on prépare au préalable un oxométallate B

B $\quad X_{12}^6 Cu_g X_h^7 O_y$

sous forme finement divisée, et on le mélange avec un mélange intime sec finement divisé de sources des constituants élémentaires d'un oxométallate A

A       $Mo_{12} X_a^1 X_b^2 X_c^3 X_d^4 S_e X_f^5 O_x$,

et on calcine le mélange à une température de 250 à 450°C.

9.  Procédé pour la préparation par oxydation catalytique en phase gazeuse de l'acide méthacrylique à partir de la méthacroléine, caractérisé en ce que l'on utilise comme catalyseur un oxyde multimétallique selon les revendications 1 à 7.

10. Procédé selon la revendication 9, caractérisé en ce que le catalyseur utilisé consiste en un catalyseur massif dont la géométrie est celle d'un cylindre creux ayant un diamètre externe et une longueur de 2 à 10 mm et une épaisseur de paroi de 1 à 3 mm.